# EUROPEAN PATENT APPLICATION

(11) **EP 1 702 615 A1**
(43) Date of publication of application: **20.09.2006**
(21) Application number: 04807786.1
(22) Date of filing: 24.12.2004
(51) Int. Cl.: A61K 31/19, A61P 3/10

(54) **GLUCOSE TOLERANCE-IMPROVING AGENT**

(30) Priority: 26.12.2003 JP 2003432770
(71) Applicant: Use-Techno Corporation, Fukuchiyama-shi, Kyoto 6200055 (JP)
(72) Inventor: MATSUYAMA, Futoshi, 6208502 (JP); SEINO, Yutaka, 6610003 (JP); FUKUSHIMA, Mitsuo, Kyoto-shi, Kyoto, 6038156 (JP)
(74) Representative: Ouzman, Beverley Nicola Claire
(86) International application number: PCT/JP2004/019430
(87) International publication number: WO 2005/063228

(57) **Abstract**

The present invention provides a glucose tolerance improving agent containing as an active ingredient at least one triterpene selected from the group consisting of corosolic acid, analogous compounds of corosolic acid, and pharmaceutically acceptable salts thereof.

## Description

### Technical Field

The present invention relates to a glucose tolerance improving agent.

### Background Art

The number of diabetic patients is increasing every year and this is becoming a serious social problem. Non-insulin-dependent diabetes mellitus, which is the most common form of diabetes, is a disease wherein decreased insulin secretion from pancreas β cells and decreased insulin sensitivity (insulin resistance) in skeletal muscle, liver, fatty tissue, etc., which are insulin target organs, together lead to deficiency of insulin action which results in hyperglycemia.

A World Health Organization (WHO) Consultation defined as diabetes a state wherein the fasting blood glucose level (glucose concentration in venous plasma) is 126 mg/dl or more, or a state wherein the blood glucose level at 2 hours after a glucose load in a glucose tolerance test is 200 mg/dl or more. It further defined as impaired glucose tolerance a state wherein the fasting blood glucose level (glucose concentration in venous plasma) is less than 140 mg/dl, and the blood glucose level (glucose concentration in venous plasma) at 2 hours after a glucose load in a 75 g oral glucose tolerance test is 140 mg/dl or more, and less than 200 mg/dl, and warned people with impaired glucose tolerance that their state could progress to diabetes or arteriosclerotic angiopathy.

At present, what are most frequently used as oral antidiabetic agents are sulfonylurea agents (SU agents), and some triterpenes such as corosolic acid are also known to suppress a blood glucose increase (e.g., Non-Patent Document 1).
Non-Patent Document 1: Japanese Pharmacology and Therapeutics, 1999, Vol.27, No.6, pp.1075-1077

### Disclosure of the Invention

### Problem to be Solved by the Invention

However, although many conventional diabetic medicines suppress a temporary blood glucose increase when a meal is taken, they are thought to be unable to improve an ability to restore a high blood glucose level to normal (hereinafter referred to as "glucose tolerance") in diabetic patients or people with impaired glucose tolerance.

For example, although SU agents suppress a blood glucose increase caused by food taking by stimulating insulin secretion from pancreas β cells, the effect lasts only temporarily, and is not maintained if the use of the agent is interrupted. In other words, SU agents are not agents which improve a patient's glucose tolerance itself, and which restore the patient to a state where normal glucose tolerance is maintained without further taking a therapeutic agent.

Therefore, when patients with non-insulin-dependent diabetes mellitus found it difficult to maintain proper blood glucose levels only through diet and exercise therapy, therapeutic agents such as SU agents often had to be taken daily, for example before meals, on a continuous basis, and this placed a considerable burden on the patients.

It is therefore an object of the present invention to provide a glucose tolerance improving agent which improves glucose tolerance and maintains normal glucose tolerance with a small number of doses.

### Means for Solving the Problem

During research on the mechanism by which triterpenes such as corosolic acid and its analogous compounds suppress a blood glucose increase, the present inventors found that these triterpenes not only have the effect of suppressing a blood glucose increase immediately after intake, but that this effect continues for several days or more. They presumed that these triterpenes have the effect of restoring an individual's reduced glucose tolerance to normal levels, and based on this finding, arrived at the present invention.

A glucose tolerance improving agent provided by the present invention contains as an active ingredient at least one triterpene selected from the group consisiting of corosolic acid, analogous compounds of corosolic acid, and pharmaceutically acceptable salts thereof. The analogous compounds of corosolic acid are preferably tormentic acid and maslinic acid due to the magnitude of their glucose tolerance improving effect.

It was already known that triterpenes such as corosolic acid and its analogous compounds could suppress a sharp rise of blood glucose resulting from, for example, food taking, and it is thought that this effect is due to promotion of glucose uptake in skeletal muscle etc. and stimulation of early insulin secretion from pancreas β cells. However, it was not known at all until now that these triterpenes can also be used as glucose tolerance improving agents that restore an individual's glucose tolerance to normal levels.

The glucose tolerance improving agent of the present invention contains the aforesaid triterpene as an active ingredient, and can be taken independently of mealtimes. Many conventional diabetic medicines often had to be taken before meals in order to prevent a sharp rise of blood glucose caused by food taking. However, as the glucose tolerance improving agent of the present invention contains the aforesaid triterpene as an active ingredient, it can be taken irrespective of mealtimes, for example either before or after meals, and the number of doses can be reduced. Therefore, it can alleviate the burden placed on a patient.

A glucose tolerance improving method provided by the present invention is one wherein the aforesaid glucose tolerance improving agent is taken by an individual and improves the individual's glucose tolerance.

### Effects of the Invention

According to the glucose tolerance improving agent of the present invention, the blood glucose suppression effect can be maintained with a small number of doses. Further, the glucose tolerance improving agent of the present invention do not easily cause side effects such as hypoglycemia, and it can be taken over a long period as a drug, a food, a drink, or an additive thereto with its high level of safety maintained.

### Brief Description of the Drawings

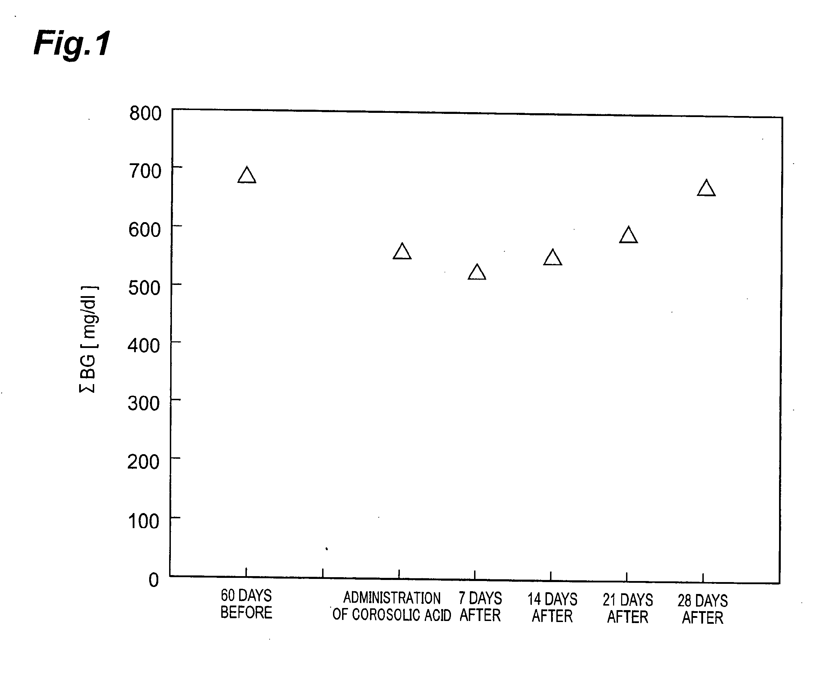

Fig. 1 is a diagram showing changes in measured ΣBG in relation to time.

### Best Modes for Carrying Out the Invention

Embodiments of the present invention will now be described in detail.

The glucose tolerance improving agent of the present invention contains as an active ingredient at least one triterpene selected from the group consisting of corosolic acid, which is represented by the following formula (1), analogous compounds of corosolic acid, and pharmaceutically acceptable salts thereof. It may consist of only any of these triterpenes, or may further contain another ingredient.

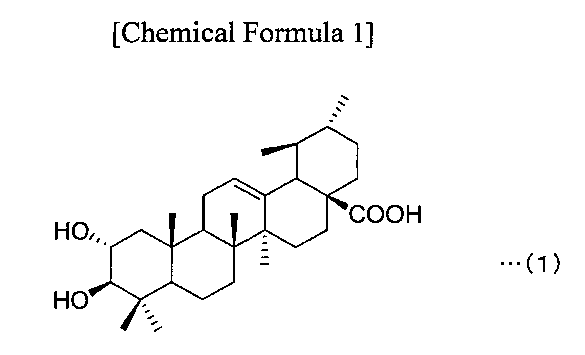

The analogous compound of corosolic acid used as an active ingredient is preferably, for example, a triterpene having a carboxyl group or a carboxylate group at the 28-position, or additionally, hydroxyl groups at the 2α- and 3β-positions. Specifically, tormentic acid, which is represented by the following formula (2), and maslinic acid, which is represented by the following formula (3), are particularly preferred.

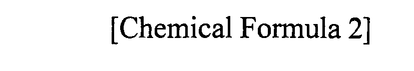

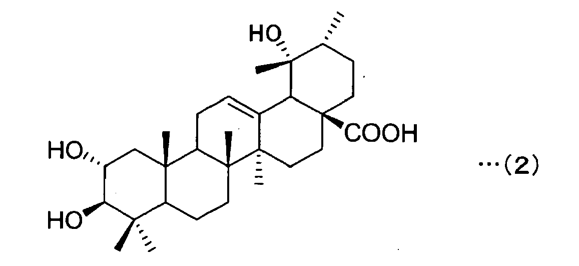

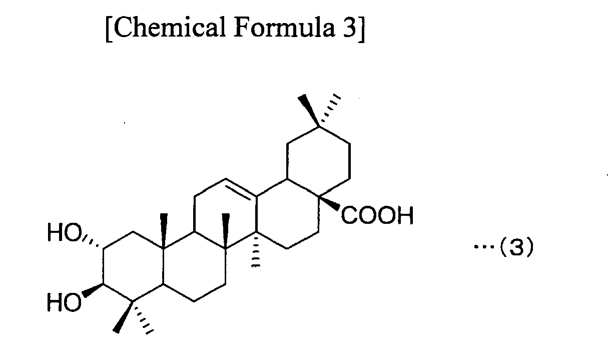

Examples of the pharmaceutically acceptable salts of these triterpenes are alkali metal salts, alkaline earth metal salts and ammonium salts. Specifically, as preferred active ingredients, there may be mentioned salts of triterpenes with sodium, potassium, calcium, magnesium, ammonia, dimethylamine, diethylamine, trimethylamine, tetramethylammonium, monoethanolamine, diethanolamine or triethanolamine.

Among these, corosolic acid has a pronounced glucose tolerance improving effect, so the glucose tolerance improving agent preferably contains corosolic acid as an active ingredient.

Many kinds of plants contain corosolic acid and its salts, so plant extracts can be used. The extracts are preferably ones obtained from the leaf of banaba (Lagerstroemia speciosa Linn. or Pers.), which contains a large amount of corosolic acid. Banaba is a variety of crape myrtle distributed throughout tropical Asia, which belongs to Myrtales Lythraceae and is also called Pride of India.

Tormentic acid and its salts can be extracted from plants such as Acaene pinnatifida, Agrimonia pilosa, Rosa roxburgii, Eriope blanchetii, Perilla frutescens, Debregeasia salicifolia, Rubus sieboldii or Tiarella polyphylla.

Maslinic acid and its salts can be extracted from plants such as loquat (Eriobotrya japonica), olive (Olea europaea) or Crataegus pinnatifida.

To obtain triterpenes, a part such as a leave or stem is cut from the living plant. Triterpenes are then extracted from the cut part, either raw or dried. Alternatively, a plant tissue such as callus tissue is grown from the cut part and made to produce triterpenes, and triterpenes are then extracted from the cultured tissue. If the culturing of a plant tissue is performed, the tissue used is preferably callus tissue derived from banaba, because corosolic acid, tormentic acid and maslinic acid can be efficiently obtained.

The solvent used for extraction of triterpenes from plants is preferably a hydrophilic solvent, for example water or an alcohol such as methanol or ethanol, but more preferably, a warm water/alcohol mixed solvent. Specifically, a suitable method is one wherein ethanol or an aqueous ethanol solution (50-80 wt% ethanol content) is added to dried pulverized banaba leaves (raw material) at 5-20 times by weight, preferably 8-10 times by weight, with respect to the raw material, the mixture is heated to reflux for extraction at a temperature from normal temperature to 90°C, preferably from about 50°C to 85°C, for a period from 30 minutes to 2 hours, and the extraction is repeated 2 or 3 times.

The triterpenes obtained from the extract can be purified, for example, using silica gel column chromatography or by recrystallization.

When there is a large amount of the extract, the following method is preferred. After suspending the extract in water, it is distributed in ether or hexane to first remove low polarity components. The aqueous layer is then successively eluted with water, methanol and acetone using Diaion HP-20 column chromatography or the like. The methanol fraction is then subjected to separation and purification using silica gel column chromatography or the like.

When trying to obtain triterpenes in high purity by separation and purification, the following method is also preferred. After acetylation of hydroxyl groups or methyl esterification of carboxyl groups in the triterpenes, purification is performed using silica gel chromatography or by recrystallization, and hydrolysis is performed to obtain desired triterpenes.

The glucose tolerance improving agent of the present invention may, in addition to an active ingredient consisting of the aforesaid triterpene, further contain an excipient for drug formulations. Preferred examples of such excipients are lactose, starch, and the like. These excipients make it possible to use the glucose tolerance improving agent in various solid or liquid formulations.

As a specific example of a glucose tolerance improving agent formulation, there may be mentioned a capsule formulation obtained by mixing 10 mg of corosolic acid and 100 mg of cornstarch, granulating the mixture, and then enclosing the granules in a gelatin capsule.

The glucose tolerance improving agent of the present invention can also be added, as an additive for food and drink, to drinks such as water, soft drinks, fruit juices, milk beverages and alcoholic beverages, and foods such as bread, noodles, rice, bean curd, dairy products, soy sauce, soybean paste and confectionery.

The glucose tolerance improving agent of the present invention can be taken irrespective of mealtimes, for example either before or after meals, to suppress a blood glucose increase caused by food taking. The timing for taking each dose and the number of doses can be determined depending on the medical condition of an individual who will take the agent, but usually, by taking it once a day, the individual's glucose tolerance can be maintained at normal levels and a blood glucose increase caused by food taking can be suppressed.

When the period for which an individual's glucose tolerance is maintained at normal levels exceeds one day, the number of doses may be further reduced. It may for example be reduced to once per 2-20 days, or preferably once per 10-20 days.

The amount of the glucose tolerance improving agent to be taken on each occasion is an amount sufficient to improve an individual's glucose tolerance, and preferably sufficient to maintain an individual's glucose tolerance at normal levels for one day or more. Specifically, the amount of triterpene which is the active ingredient may be 0.1-1000 mg per 60 kg of body weight, but preferably 1-20 mg.

An individual who should be given the glucose tolerance improving agent of the present invention may be a human or an animal. The human or the animal may or may not have diabetes. If the human or the animal has diabetes, the diabetes is preferably of the non-insulin-dependent type.

As the glucose tolerance improving agent of the present invention has a low risk of causing hypoglycemia, it is suitable for humans and animals whose glucose tolerance has been reduced, but who have not yet had diabetes. For example, in the case of humans, those who have impaired glucose tolerance according to the WHO diagnostic criteria can take this agent.

The glucose tolerance improving method of the present invention is one wherein the aforesaid glucose tolerance improving agent is taken by an individual with reduced glucose tolerance and improves the individual's glucose tolerance, and the individual may be a human or an animal. In this method, active ingredients, individuals who should take the agent, and the number of doses may be as specified above for the glucose tolerance improving agent.

### Examples

The present invention will now be explained in greater detail referring to the following examples, with the understanding that these examples are in no way limitative on the present invention.

### (Glucose tolerance improving effect of corosolic acid administration in a dog)

A dog (beagle) was used as the test animal. 20 mg/kg of body weight of purified corosolic acid extracted from banaba leaves was administered to the animal, and the glucose tolerance improvement effect was evaluated.

The glucose tolerance was evaluated by performing glucose tolerance tests according to the procedure described in (1)-(3) below. A glucose tolerance test was performed 60 days before corosolic acid administration, immediately after corosolic acid administration, and at 7 days, 14 days, 21 days and 28 days after corosolic acid administration. At time points other than immediately after corosolic acid administration, the glucose tolerance test was performed immediately after the same dose of D-sorbitol was orally administered as a placebo instead of corosolic acid.
(1) 75 g of glucose is administered orally to the dog.
(2) Blood is collected from the dog's vein at 0 minute, 30 minutes, 60 minutes, 90 minutes, 120 minutes, 180 minutes and 240 minutes after glucose administration, and blood glucose levels of the collected blood are measured using GLUTEST SENSOR (trade name, manufactured by SANWA KAKAGU KENKYUSHO CO., LTD).
(3) The total of the 7 blood glucose levels measured in (2) above is represented by ΣBG [mg/dl]. In this model, if ΣBG is low, it shows that glucose tolerance is improved, and that a blood glucose increase caused by food taking is suppressed.

Fig. 1 is a diagram showing the changes in measured ΣBG in relation to time. As shown in Fig.1, ΣBG has lower values for 28 days after corosolic acid adminisration than before corosolic acid adminisration, and the improved glucose tolerance continued during this period without taking any more corosolic acid.

## Claims

1. A glucose tolerance improving agent containing as an active ingredient at least one triterpene selected from the group consisting of corosolic acid, analogous compounds of corosolic acid, and pharmaceutically acceptable salts thereof.

2. The glucose tolerance improving agent according to claim 1, wherein said analogous compound of corosolic acid is tormentic acid or maslinic acid.

3. The glucose tolerance improving agent according to claim 1 or 2, wherein said glucose tolerance improving agent can be taken independently of mealtimes.

4. A glucose tolerance improving method, wherein the glucose tolerance improving agent according to any one of claims 1 to 3 is taken by an individual.
